# EUROPÄISCHE PATENTSCHRIFT

(11) **EP 1 515 740 B1**
(45) Veröffentlichungstag und Bekanntmachung des Hinweises auf die Patenterteilung: **23.08.2006**
(21) Anmeldenummer: 03732406.8
(22) Anmeldetag: 19.05.2003
(51) Int. Cl.: A61K 38/08, A61K 47/48

(54) **FLÜSSIGE ZUBEREITUNG ENTHALTEND OLIGOPEPTIDE UND VERETHERTES CYCLODEXTRIN**
AQUEOUS PREPARATION CONTAINING OLIGOPEPTIDES AND ETHERIFIED CYCLODEXTRIN
PREPARATION LIQUIDE CONTENANT DES OLIGOPEPTIDES ET DE LA CYCLODEXTRINE ETHERIFIEE

(30) Priorität: 24.06.2002 DE 10228049
(43) Veröffentlichungstag der Anmeldung: 23.03.2005
(73) Patentinhaber: Merck Patent GmbH, 64293 Darmstadt (DE)
(72) Erfinder: LINDENBLATT, Hiltrud, 63329 Egelsbach (DE); ZOBEL, Hans-Peter, 65439 Floersheim (DE)
(86) Internationale Anmeldenummer: PCT/EP2003/005224
(87) Internationale Veröffentlichungsnummer: WO 2004/000344

(56) Entgegenhaltungen:
- WO-A-00/62793
- WO-A-85/02767
- WO-A-91/11200
- US-A- 6 001 961
- KAMM, W. ET AL: "Evaluation of absorption enhancement for a potent cyclopeptidic.alpha..nu..beta.3-antagonist in a human intestinal cell line (Caco-2)" EUROPEAN JOURNAL OF PHARMACEUTICAL SCIENCES (2000), 10(3), 205-214 , XP002251179

## Beschreibung

Die vorliegende Erfindung betrifft eine wässrige pharmazeutische Zubereitung von Oligopeptiden der Formel I, enthaltend ein Oligopeptid und ein verethertes β-Cyclodextrin mit einer Wasserlöslichkeit von größer als 1,8 mg / ml Wasser sowie die Herstellung der wässrigen pharmazeutischen Zubereitung.

Die in der erfindungsgemäßen Zubereitung enthaltenen Oligopeptide sind Cyclopeptide der Formel I

Cyclo-(n-Arg-nGly-nAsp-nD-nE)

worin
- D und E: jeweils unabhängig voneinander Gly, Ala, β-Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-Hal-Phe, homoPhe, Phg, Pro, Pya, Ser, Thr, Tia, Tic, Trp, Tyr oder Var, wobei die genannten Aminosäurereste auch derivatisiert sein können,
- R: Alkyl mit 1-18 C-Atomen,
- Hal: F, Cl, Br, I,
- Ac: Alkanoyl mit 1-10 C-Atomen, Aroyl mit 7-11 C-Atomen oder Aralkanoyl mit 8-12 C-Atomen,
- n: ein Hydrogenatom oder einen Alkylrest R, Benzyl oder einen Aralkyl-Rest mit 7-18 C-Atomen an der alpha-Aminofunktion des entsprechenden Aminosäurerestes
bedeuten,
mit der Bedingung, dass mindestens ein Aminosäurerest über einen Substituenten n verfügt, wobei n R bedeutet, und wobei, sofern es sich um Reste optisch aktiver Aminosäuren und Aminosäurederivate handelt, sowohl die D- als auch die L- Formen eingeschlossen sind, sowie deren physiologisch unbedenkliche Salze.

Die Oligopeptide der Formel I sind in EP 0 770 622 A2 beschrieben. Hinsichtlich des Bedeutungsinhalts der in Formel I enthaltenen Aminosäuren und Substituenten sowie der Herstellung der Peptide wird auf diese Druckschrift verwiesen.

Die Oligopeptide der Formel I wirken als Integrin-Inhibitoren, wobei sie insbesondere die Wechselwirkungen der β₃- oder β₅-Integrin-Rezeptoren mit endogenen Liganden hemmen. Die Verbindungen zeigen Wirksamkeit gegenüber den Integrinen αᵥβ₃, αᵥβ₅, αᵥβ₆ und α_{II}β₃ aber auch gegenüber αᵥβ₁-, und αᵥβ₈-Rezeptoren. Besondere Bedeutung kommt dabei der Blockade der αᵥβ₃ und αᵥβ₆-Rezeptoren zu. Besonders erwähnt sei hierbei die Verhinderung der Stimulation von αᵥβ₃-Rezeptoren durch den endogene Liganden Fibrinogen.

Die beschriebenen Interaktionen führen insbesondere zu einer Inhibition der Angiogenese, so dass die Oligopeptide zur Krebstherapie geeignet sind. Besonders erwähnt sei hierbei das Oligopeptid Cilengitide, ein cyklisches Pentapeptid mit der chemischen Bezeichnung Cyclo-(Arg-Gly-Asp-D-Phe-NMe-Val). Cilengitide befindet sich bereits in Phase II der klinischer Prüfung zur Behandlung von Krebserkrankungen.

Wie andere Peptide werden auch die Oligopeptide der Formel I vorzugsweise parenteral als wässrige Lösung verabreicht. Zur therapeutischen Anwendbarkeit sind daher wässrige Lösungen der Oligopeptide erforderlich. Die hierzu eingesetzten wässrigen Lösungen der Oligopeptide sollten den jeweiligen Therapieerfordernissen angepasst sein, insbesondere sollten sie den Wirkstoff in der Therapie erforderlichen Menge enthalten und eine hinreichende Lagerstabilität aufweisen.

Die Behandlung von Tumorerkrankungen erfordert die parenterale Verabreichung von relativ großen Wirkstoffmengen. Die Oligopeptide sind aufgrund ihrer Peptidstruktur in Wasser relativ gut löslich. Trotzdem ergeben sich aufgrund der relativ hohen zur Therapie erforderlichen Wirkstoffmengen relativ hohe Volumina an Wirkstofflösung, die parenteral zu verabreichen sind. Diese können dann nicht mehr einfach injiziert werden, sondern müssen infundiert werden.

Cilengitide beispielsweise weist in physiologischer Kochsalzlösung eine Sättigungslöslichkeit von cirka 19 mg/ml auf, und kann daher für die therapeutische Anwendung in physiologischer Kochsalzlösung gelöst sicher in einer Konzentration von 15 mg/ml parenteral verabreicht werden. Ist zur Therapie mit Cilengitide beispielsweise eine Dosierung von 1500 mg erforderlich, ergibt sich ein zu verabreichendes Volumen von 100 ml. Volumina in dieser Größenordnung können nicht mehr ohne weiteres injiziert werden und müssen nachteilhaft infundiert werden.

Um das jeweils zu verabreichende Volumen an Wirkstofflösung zu vermindern, ist es wünschenswert den Wirkstoffanteil in der jeweiligen wässrigen Lösung zu erhöhen. Wie auch andere Peptide ist die Löslichkeit der Oligopeptide von dem pH-Wert des jeweiligen Lösungsmittels abhängig. Als löslichkeitserhöhende Maßnahme in Frage kommt daher insbesondere die Einstellung des pH-Wertes des wässrigen Lösungmittels auf einen Wert, in dem das Oligopeptid eine höhere Löslichkeit aufweist. Die hierzu erforderlichen pH-Werte liegen jedoch in einem unphysiologischen Bereich, was im Hinblick auf eine parenterale Verabreichung äußerst kritisch zu sehen ist. Weiterhin führt ein vom physiologischen pH-Wert stark abweichender pH-Wert zumeist zu beschleunigtem Peptidabbau in wässriger Lösung, so dass derartige Lösungen auch nicht hinreichend lagerstabil sind.

Umfangreiche Versuche die Löslichkeit der Oligopeptide zu erhöhen verliefen ohne den gewünschten Erfolg. Erfolglos wurde beispielsweise versucht die Löslichkeit von Cilengitide über einen Zusatz an physiologisch verträglichen organischen Lösungsmitteln wie Ethanol oder Propandiol zu verbessern. Ebenso ergab der Zusatz von Tensiden wie Cremophor und Polysorbat 80 keine wesentliche Verbesserung der Löslichkeit an Cilengitide. Durch Gemische an Citronensäure, Phosphatpuffer und Tensiden konnte die Löslichkeit an Cilengitide zwar erhöht werden, doch waren diese Lösungen nicht lagerstabil.

Aus EP 0149 197 ist bekannt, dass Cyclodextrinether die Wasserlöslichkeit von schwer wasserlöslichen Arzneistoffen erhöhen können. Hierbei sollen Einschlussverbindungen gebildet werden, wobei die Arzneistoffe in den hydrophoben Hohlraum des Cyclodextrin-Ringsystems eindringen. Eine Voraussetzung für die Fähigkeit der Arzneistoffe in den Hohlraum ist, dass diese auch in den Hohlraum hinein passen. Die Arzneistoffe dürfen daher auch nicht eine bestimmte räumliche Ausdehnung überschreiten. Alle in EP 0149 197 angeführten Arzneistoffe sind niedrigmolekulare chemische Verbindungen und in Wasser schwer löslich. Demgegenüber sind die in Frage stehenden Wirkstoffe in Wasser relativ gut lösliche Peptide, die zudem noch eine vergleichsweise hohes Molekulargewicht und damit einhergehend eine größere räumliche Ausdehnung aufweisen.

Es war daher Aufgabe der vorliegenden Erfindung eine zur parenteralen Verabreichung geeignete wässrige Zubereitung mit einem erhöhten Anteil an Oligopeptiden der Formel I zur Verfügung zu stellen. Die Zubereitung sollte keine toxikologisch bedenklichen Hilfsstoffe enthalten und über längere Zeit stabil sein.

Überraschenderweise konnte eine diesen Anforderungen entsprechende Zubereitung mit einer Lösung gefunden werden, die neben einem Oligopeptid der Formel I ein β-Cyclodextrinether mit einer Wasserlöslichkeit von über 1,8 mg/ml enthält.

Die erfindungsgemäße Zubereitung kann bei Kühlschranktemperatur (2-8°C) und sogar bei Raumtemperatur (25°C, 60% r.F.) über einen Zeitraum von mindestens 6 Monaten stabil gelagert werden. Überraschenderweise ist die erfindungsgemäße Zubereitung auch bei höheren Temperaturen und Luftfeuchtigkeiten, beispielsweise bei einer Temperatur von 30°C, 60% r.F. über 3 Monate und von 40°C und 75% r.F. über 4 Wochen stabil lagerbar.

β-Cydodextrin ist ein α-1,4-verknüpftes ringförmiges Oligosaccharid aus 7 Glucoseeinheiten das bei Raumtemperatur in Wasser eine Sättigungslöslichkeit von 1,8 mg/ml aufweist. Jede der (Anhydro)-Glucoseeinheiten von β-Cyclodextrin enthält in 2-, 3-, und 6-Stellung freie Hydroxylgruppen, die jeweils verethert werden können. Werden die freien Hydroxylgruppen an den Glucoseeinheiten vollständig oder teilweise mit einer oder mehreren polaren, d.h. gut wasserlöslichen, Gruppe/n enthaltenden Alkylgruppen, wie beispielsweise einer Hydroxyalkylgruppe, verethert, entstehen veretherte β-Cyclodextrine mit gegenüber reinem β-Cyclodextrin erhöhter Wasserlöslichkeit. Geeignete, die Wassedöslichkeit erhöhende Hydroxyalkylgruppen sind beispielsweise Hydroxyethyl- oder Hydroxypropylgruppen, die durch Reaktion des β-Cyclodextrins mit den entsprechenden Alkylenoxiden wie Ethylenoxid oder Propylenoxid in das β-Cyclodextrin eingeführt werden können.
Vorzugsweise sind die enthaltenen Ethersubstituenten Hydroxyethyl- und/oder Hydroxypropyl-gruppen.

Bevorzugt enthält die wässrige pharmazeutische Zubereitung partiell verethertes β-Cyclodextrin, d.h. β-Cyclodextrin, in dem nur ein Teil der Hydroxylgruppen der Anhydroglucoseeinheiten verethert vorliegen.

Je nach Menge an im Verhältnis zum β-Cyclodextrin zur Veretherung eingesetztem Alkylenoxid entstehen veretherte β-Cyclodextrine mit unterschiedliche hohem Substitutionsgrad. Der auf die Ethersubstitution bezogenene Substitutionsgrad wird nachfolgend als molarer Substitutionsgrad (MS) ausgedrückt und bezeichnet die je Mol (Anhydro)-glucoseeinheit eingesetzte Molmenge an Alkylenoxid.

Erfindungsgemäß weisen die in der wässrigen pharmazeutischen Zubereitung enthaltenen partiell veretherten β-Cyclodextrine einen molaren Substitutionsgrad zwischen 0,2 und 10 auf. Bevorzugt sind veretherte β-Cyclodextrine mit einem molaren Substitutionsgrad zwischen 0,2 und 2, besonders bevorzugt mit einem molaren Substitutionsgrad zwischen 0,5 und 0,8 und ganz besonders bevorzugt mit einem molaren Substitutionsgrad von cirka 0,58 - 0,73.

Die erfindungsgemäße wässrige Lösung kann als Oligopeptid jedes der von der oben stehenden allgemeinen Formel I umfassten Oligopeptide enthalten. Bevorzugt enthält die wässrige pharmazeutische Zubereitung als Oligopeptid Cyclo-(NMeArg-Gly-Asp-D-Phe-Val), Cyclo-(Arg-Gly-Asp-DPhe-NMeVal), Cyclo-(Arg-NMeGly-Asp- DPhe-Val), Cyclo-(Arg-Gly-NMeAsp-DPhe-Val) oder Cyclo-(Arg-Gly-Asp-NMeDPhe-Val). Besonders bevorzugt ist Cilengitide enthalten. Clilengitide hat, wie bereits oben erwähnt, die chemische Bezeichnung Cyclo-(Arg-Gly-Asp-D-Phe-NMe-Val).

Soweit durch die osmotischen Eigenschaften des Oligopeptids und durch das Cyclodextrin die wässrige Zubereitung nicht bereits isotonisch ist, kann weiterhin ein Isotonisierungsmittel, bevorzugt ein physiologisch verträgliches Salz, wie beispielsweise Natrium- oder Kaliumchlorid, oder ein physiologisch verträgliches Polyol oder ein Zucker, wie beispielsweise Glucose oder Glycerin oder Mannitol, in einer zur Isotonisierung erforderlichen Menge enthalten sein.

Weiterhin kann die erfindungsgemäßen wässrige Zubereitung weitere physiologisch verträgliche Hilfsstoffe, wie z.B. Antioxidantien wie Ascorbinsäure oder Glutathion, Konservierungsmittel wie Phenol, m-Cresol, Methyl- oder Propylparaben, Chlorbutanol, Thiomersal oder Benzalkoniumchlorid, oder weitere Stabilisatoren, Gerüstbildner und Lösungsvermittler wie Polyethylenglykole (PEG), z.B. PEG 3000, 3350, 4000 oder 6000 oder Dextrane enthalten.

Darüber hinaus können in der erfindungsgemäßen wässrigen Zubereitung Puffer enthalten sein, wobei grundsätzlich alle physiologisch verträglichen Substanzen einsetzbar sind, die zur Einstellung des gewünschten pH-Wertes geeignet sind. Soweit eine Puffersubstanz enthalten ist diese in einer Konzentration von 5 mMol/l bis 50 mMol/l, vorzugsweise in einer Konzentration von 10 bis 20 mMol/l enthalten. Als Puffer bevorzugt sind Citratpuffer oder Phosphatpuffer. Geeignete Phosphatpuffer sind Lösungen der Mono- und/oder Di-Natrium- und Kaliumsalze der Phosphorsäure, wie Dinatriumhydrogenphosphat oder Kaliumdihydrogenphosphat, sowie Mischungen der Natrium- und Kaliumsalze, wie beispielsweise Mischungen aus Dinatriumhydrogenphosphat und Kaliumdihydrogenphosphat.

Vorteilhaft weist die wässrige Zubereitung einen pH-Wert von 5 bis 8, bevorzugt einen pH-Wert von 5,6 bis 7,4, besonders bevorzugt einen pH-Wert von 6 bis 7,2 auf. Die Osmolalität beträgt vorzugsweise 250 bis 350 mOsmol/kg. Die wässrige Zubereitung kann somit weitgehend schmerzfrei direkt intravenös oder auch intraarteriell verabreicht werden.

Nach einer bevorzugten Ausführungsform der Erfindung enthält die wässrige pharmazeutische Zubereitung 20 bis 120 mg/ml Cilengitide und 15 bis 25 Gew.-% Hydroxypropyl-β-Cyclodextrin mit einem molaren Substitutionsgrad von 0,5 bis 0,8.

Nach einer besonders bevorzugten Ausführungsform der Erfindung enthält die wässrige pharmazeutische Zubereitung cirka 80 mg/ml Cilengitide und cirka 20 Gew.-% 2-Hydroxypropyl-β-Cyclodextrin mit einem molaren Substitutionsgrad von cirka 0,58-0,73.

Die wässrige Zubereitung kann hergestellt werden, indem die in der Zubereitung enthaltenen Stoffe nacheinander in Wasser gelöst werden. Zweckmäßigerweise wird zunächst der Cyclodextrinether in Wasser gelöst und anschließend das Oligopeptid sowie ggf. die weiteren Hilfsstoffe hinzu gefügt. Gegenstand der Erfindung ist daher auch ein Verfahren zur Herstellung der erfindungsgemäßen wässrigen pharmazeutischen Zubereitung, das dadurch gekennzeichnet ist, dass man zunächst den β-Cyclodextrinether in Wasser löst und anschließend den Wirkstoff sowie ggf. die weiteren Hilfsstoffe zufügt.

Soweit dies erforderlich ist, wird die das jeweilige Oligopeptid, den β-Cyclodextrinether sowie gegebenenfalls die weiteren Hilfsstoffe enthaltende Lösung noch auf einen pH-Wert von 5 bis 8 eingestellt. Anschließend wird sterilfiltriert.

Die Beispiele, ohne darauf beschränkt zu sein, erläutern die Erfindung.

### Beispiel 1

### Sättigungslöslichkeiten der Oligopeptide am Beispiel Cilengitide

Zur Bestimmung der Sättigungslöslichkeiten wurde das Oligopeptid bei Raumtemperatur im jeweils angeführten Lösungsmittel über 1 Stunde gerührt. Die Ergebnisse sind in Tabelle 1 angeführt.

**Tabelle 1**

| Rezeptur | Cilengitide [mg/ml] | pH-Wert |
|---|---|---|
| Wasser für Injektionszwecke | 14,67 | 6,65 |
| Ethanol/Wasser (10 Vol.% Ethanol) | 6,68 | 6,63 |
| Ethanol/Wasser (30 Vol.% Ethanol) | 3,91 | 6,90 |
| Propandiol/Wasser (10 Vol.% Propandiol) | 13,44 | 6,79 |
| Propandiol/Wasser (30 Vol.% Propandiol) | 12,54 | 7,01 |
| Propandiol/Wasser (50 Vol.% Propandiol) | 8,23 | 7,17 |
| Phosphatpuffer pH 1 | 106,10 | 1,23 |
| Phosphatpuffer pH 2 | 65,47 | 3,99 |
| Phosphatpuffer pH 3 | 22,48 | 4,79 |
| Phosphatpuffer pH 5 | 18,64 | 5,50 |
| Phosphatpuffer pH 7 | 17,98 | 6,98 |
| 0,9% NaCl-Lösung in Wasser | 19,21 | 6,63 |
| Citronensäure-/Phosphatpuffer, pH 2,5 | 83,86 | 3,85 |
| Citronensäure-/Phosphatpuffer, pH 3 | 61,89 | 3,98 |
| Citronensäure-/Phosphatpuffer, pH 3,5 | 50,03 | 4,24 |
| Citronensäure-/Phosphatpuffer, pH 3 mit 0,5% Polysorbat 80 VS | 83,19 | 4,14 |
| Citronensäure-/Phosphatpuffer, pH 3 mit 0,2% Cremophor RH 40 | 73,14 | 4,09 |
| Citronensäure-/Phosphatpuffer, pH 3 mit 30 % Glycerin | 71,48 | 4,33 |
| Citronensäure-/Phosphatpuffer, pH 3 mit 30 % Glycerin u. 0,5% Polysorbat 80 VS | 72,67 | 4,32 |

| | | |
|---|---|---|
| * pH-Wert von 80 mg Cilengitide in 20% 2-Hydroxypropyl-β-cyclodextrin: 7,02 | | |

Die Ergebnisse zeigen, dass sich die Sättigungslöslichkeit des Oligopeptids in Wasser durch Zusatz der Alkohole Ethanol und Propandiol nicht erhöhen ließ sondern verschlechterte. Hingegen ergeben verschiedene Puffergemische im sauren Bereich eine deutliche Erhöhung der Sättigungslöslichkeit. Werden sauren Lösungen, die eine erhöhte Löslichkeit für das Oligopeptid aufweisen, Tenside zugefügt, führt dies zu keiner weiteren deutlichen Löslichkeitserhöhung. Wird den sauren Lösungen anstatt der Tenside oder zusätzlich zu diesen Glycerin zugegeben, ist sogar eine Abnahme der Löslichkeit zu verzeichnen.

### Beispiel 2

### Stabilität ausgewählter Zusammensetzungen von Beispiel 1 mit hoher Sättigungslöslichkeit für das Oligopeptid

Ausgewählte Zusammensetzungen, in denen Cilengitide eine hohe Sättigungslöslichkeit aufweist, wurden bei 25°C, 60% r.F. sowie bei 40°C und 75% r.F. für 8 bzw. 26 Wochen eingelagert und zu Beginn (Start) sowie nach Ablauf der Lagerung hochdruckflüssigkeitschromatographisch (HPLC-UV) auf lhren Gehalt an Cilengitide untersucht. Die Ergebnisse sind Tabelle 2 dargestellt.

**Tabelle 2**

| Zusammensetzung | Konzentration Cilengitide [mg/ml] | Gehalt Cilengitide [%] | | |
|---|---|---|---|---|
| | | Start | 8 Wochen 25°C/60% r.F. | 26 Wochen 40°C/75%r.F. |
| Citronensäure Phosphatpuffer pH 3* | 60 | 98,45 | 66,6 | nicht bestimmt |
| Citronensäure Phosphatpuffer pH 3 + 0,5% Polysorbat 80 VS* | 60 | 98,43 | 66,77 | nicht bestimmt |
| Citronensäure Phosphatpuffer pH 2,5* | 60 | 98,57 | 58,07 | nicht bestimmt |
| Citronensäure Phosphatpuffer pH 3 + 0,2% Cremophor RH 40* | 60 | 98,97 | 67,07 | nicht bestimmt |
| Citronensäure Phosphatpuffer pH 3, isotonisiert mit NaCl** | 60 | 98,38 | 70,72 | nicht bestimmt |
| Citronensäure Phosphatpuffer pH 3 + 0,2% Cremophor RH 40, isotonisiert mit NaCl** | 60 | 98,9 | 70,82 | nicht bestimmt |
| Natriumchlorid isotonisch* | 15 | 98,87 | 99,2 | 98 |
| Citronensäure Phosphatpuffer pH 7, NaCl** | 15 | 98,8 | 99 | 96,6 |
| NaCl, Phosphatpuffer pH 7** | 15 | 98,47 | 98,6 | 95 |

| | | | | |
|---|---|---|---|---|
| * Rezeptur aus Tabelle 1 | | | | |
| ** zusätzliche Rezeptur zu Tabelle 1 | | | | |

Keine der Zubereitungen mit Konzentrationen von 60 mg/ml Cilengitide zeigt eine ausreichende Stabilität. Zusätze von Cremophor RH 40 bzw. Polysorbat 80 VS führen gegenüber den reinen Pufferlösungen zu keiner besseren Stabilität. Die drei getesteten Zubereitungen mit 15 mg/ml Cilengitide zeigen deutlich bessere Stabilitäten, wobei die isotonische NaCl-Lösung am stabilsten ist.

### Beispiel 3

### Sättigungslöslichkeiten der Oligopeptide bei Zusatz von β-Cyclodextrinethem

Analog zu Beispiel 1 wurde am Beispiel Cilengitide der Einfluss ein Zusatzes von β-Cyclodextrinethern (MS 0,63) auf die Sättigungslöslichkeiten der Oligopeptide bestimmt. Die Ergebnisse sind in Tabelle 3 dargestellt.

**Tabelle 3**

| Rezeptur | Konzentration Cilengitide [mg/ml] | pH-Wert |
|---|---|---|
| Wasser/2-Hydroxypropyl-β-Cyclodextrin (20%) | > 90 | 7,02 (80 mg Cilengitide in 20% 2-Hydroxypropyl-β-Cyclodextrin) |
| Wasser/2-Hydroxypropyl-β-Cyclodextrin (15%) | > 60 | |
| Wasser/2-Hydroxypropyl-β-Cyclodextrin (10%) | > 40 | |

Die Ergebnisse zeigen eine deutliche Erhöhung der Löslichkeit von Cilengitide durch Zugabe der β-Cyclodextrinether. Im Gegensatz zu den in Beispiel 1 geprüften Zusätzen ist die Löslichkeitserhöhung direkt proportional zur Konzentration an β-Cyclodextrinether.

### Beispiel 4

Wässrige Zubereitung enthaltend:
200 mg 2-Hydroxypropyl-β-Cyclodextrin (MS 0,63)
80 mg Cilengitide
ad 1 ml Wasser für Injektionszwecke

Die angegebene Menge 2-Hydroxypropyl-β-Cyclodextrin wurde unter Rühren in cirka 90 % der angegebenen Menge Wasser für Injektionszwecke gelöst, die angegebene Menge Oligopeptid zugefügt und, nach Erhalt einer klaren Lösung, das restliche Lösungsmittel zugegeben. Die erhaltene Lösung wurde sterilfiltriert, in 6 ml Vials mit je 2 ml Lösung abgefüllt, mit Stopfen verschlossen und gebördelt.

### Beispiel 5

### Vergleichende Stabilitätsuntersuchung von Zubereitungen enthaltend ein Oligopeptid in isotonischer Kochsalzlösung oder in β-Cyclodextrinetherlösung

Die Zubereitung gemäß Beispiel 4 sowie eine in analoger Weise hergestellte Zubereitung enthaltend 15mg/ml Cilengitide in isotonischer Kochsalzlösung (0,9 % NaCl) wurden in Haltbarkeitsstudien überprüft. Hierzu wurden die wässrigen Zubereitungen bei verschiedenen Temperaturen eingelagert, zu bestimmten Zeiten ausgelagert und mit geeigneten analytischen Methoden untersucht. Mögliche Instabilitäten äußern sich bei Oligopeptiden in wässriger Lösung vornehmlich in der Bildung von Umlagerungs- und Hydrolyseprodukten. Im Fall von Cilengitide tragen die Zersetzungsprodukte noch die gleichen Chromophore und können wie der Ausgangsstoff mittels HPLC-UV erfasst werden. Die Ergebnisse sind in den Tabellen 4 bis 6 dargestellt.

**Tabelle 4: Stabilitätsdaten bei 2-8°C, 26 Wochen**

| Zusammensetzung | Konzentration Cilengitide [mg/ml] (Start) | Cilengitide [%] | Verunreinigung 1 [%]* | Verunreinigung 2 [%] | Verunreinigung 3 [%] |
|---|---|---|---|---|---|
| isotonische NaCl | 15 mg/ml | 100,67 | 0,48 (Start 0,43) | < 0,05 | < 0,05 |
| 2-Hydroxypropyl-β-cyclodextrin | 80 mg/ml | 99,13 | < 0,05 | < 0,05 | < 0,05 |

| | | | | | |
|---|---|---|---|---|---|
| *Verunreinigung 1 ist mit max. 2% spezifiziert. | | | | | |

**Tabelle 5: Stabilitätsdaten bei 25°C/60 % r.F., 26 Wochen**

| Rezeptur | Konzentration Cilengitide [mg/ml] (Start) | Cilengitide [%] | Verunreinigung 1 [%] | Verunreinigung 2 [%] | Verunreinigung 3 [%] |
|---|---|---|---|---|---|
| isotonische NaCl | 15 mg/ml | 101,26 | 0,84 (Start 0,43) | 0,13 | <0,05 |
| 2-Hydroxypropyl-β-cyclodextrin | 80 mg/ml | 96,56 | 0,39 | 0,14 | <0,05 |

**Tabelle 6: Stabilitätsdaten bei 30°C/60 % r.F., 26 Wochen**

| Rezeptur | Konzentration Cilengitide [mg/ml] Start | Cilengitide [%] | Verunreinigung 1 [%] | Verunreinigung 2 [%] | Verunreinigung 3 [%] |
|---|---|---|---|---|---|
| isotonische NaCl | 15 mg/ml | 100,82 | 1,16 (Start 0,43) | 0,24 | 0,06 |
| 2-Hydroxypropyl-β-cyclodextrin | 80 mg/ml | 99,37 | 0,74 | 0,25 | 0,08 |

Obwohl das Oligopeptid in der Zubereitung gemäß Beispiel 3 in mehr als 5-fach höheren Konzentration als in der Zubereitung in isotonischer Kochsalzlösung enthalten ist, weist die den β-Cycoldetrinether enhaltende Zubereitung gemäß Beispiel 3 eine ähnlich hohe Stabilität wie die Zubereitung in isotonischer Kochsalzlösung auf.

### Analytische Testmethoden:

### Aussehen

Die hergestellten Zubereitungen wurden visuell mit Hilfe einer Lichtquelle vor einer Dunkelwand gemäß Ph.Eur. auf Partikel untersucht.

### Gehalts- und Reinheitsbestimmung von Cilengitide

Die Gehalts- und Reinheitsbestimmung erfolgte mit Hilfe einer HPLC-UV Methode bei einer Wellenlänge von 215 nm. Für die Trennung wurde eine RP 18-Phase verwendet. Als Elutionsmittel kam ein Puffer mit pH 3,6 zum Einsatz bestehend aus Natriumdihydrogenphosphat und Phosphorsäure, der zu gleichem Anteil mit Acetonitril gemischt wurde. Es wurde eine Gradientenelution mit unterschiedlichem Anteil an zusätzlichem Acetonitril durchgeführt.

## Patentansprüche

1. Wässrige pharmazeutische Zubereitung von Oligopeptiden, enthaltend ein Oligopeptid der Formel I
Cyclo-(n-Arg-nGly-nAsp-nD-nE) (I)
worin
D und E jeweils unabhängig voneinander Gly, Ala, β-Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-Hal-Phe, homoPhe, Phg, Pro, Pya, Ser, Thr, Tia, Tic, Trp, Tyr oder Val, wobei die genannten Aminosäurereste auch derivatisiert sein können,
R Alkyl mit 1-18 C-Atomen,
Hal F, Cl, Br, I,
Ac Alkanoyl mit 1-10 C-Atomen, Aroyl mit 7-11 C-Atomen oder Aralkanoyl mit 8-12 C-Atomen,
n ein Hydrogenatom oder einen Alkylrest R, Benzyl oder einen Aralkyl-Rest mit 7-18 C-Atomen an der alpha-Aminofunktion des entsprechenden Aminosäurerestes
bedeuten,
mit der Bedingung, daß mindestens ein Aminosäurerest über einen Substituenten n verfügt, wobei n R bedeutet,
und wobei, sofern es sich um Reste optisch aktiver Aminosäuren und Aminosäurederivate handelt, sowohl die D- als auch die L- Formen eingeschlossen sind, sowie deren physiologisch unbedenkliche Salze
und ein verethertes β-Cyclodextrin mit einer Wasserlöslichkeit von größer als 1,8 mg / ml Wasser

2. Wässrige pharmazeutische Zubereitung gemäß Anspruch 1, **dadurch gekennzeichnet, dass** als verethertes β-Cyclodextrin partiell verethertes β-Cyclodextrin enthalten ist

3. Wässrige pharmazeutische Zubereitung gemäß Anspruch 1 oder 2, **dadurch gekennzeichnet, dass** die Ethersubstituenten im veretherten β-Cyclodextrin Hydroxyethyl- und/oder Hydroxypropyl-gruppen sind

4. Wässrige pharmazeutische Zubereitung gemäß einem oder mehreren der Ansprüche 1 bis 3, **dadurch gekennzeichnet, dass** das veretherte β-Cyclodextrin einen molaren Substitutionsgrad zwischen 0,2 und 10 aufweist

5. Wässrige pharmazeutische Zubereitung gemäß Anspruch 4, **dadurch gekennzeichnet, dass** das partiell veretherte β-Cyclodextrin bezogen auf die Ethersubstituenten einen molaren Substitutionsgrad zwischen 0,2 und 2 aufweist

6. Wässrige pharmazeutische Zubereitung gemäß Anspruch 4, **dadurch gekennzeichnet, dass** das partiell veretherte β-Cyclodextrin bezogen auf die Ethersubstituenten einen molaren Substitutionsgrad zwischen 0,5 und 0,8 aufweist

7. Wässrige pharmazeutische Zubereitung gemäß einem oder mehreren der Ansprüche 1 bis 6, **dadurch gekennzeichnet, dass** das Oligopeptid Cilengitide ist

8. Wässrige pharmazeutische Zubereitung gemäß einem oder mehreren der Ansprüche 1 bis 7, **dadurch gekennzeichnet, dass** weiterhin ein Isotonisierungsmittel in einer zur Isotonisierung erforderlichen Menge enthalten ist

9. Wässrige pharmazeutische Zubereitung gemäß einem oder mehreren der Ansprüche 1 bis 8, **dadurch gekennzeichnet, dass** diese einen pH-Wert von 5 bis 8, vorzugsweise einen pH-Wert von 5,6 bis 7,4 aufweist

10. Wässrige pharmazeutische Zubereitung gemäß Anspruch 9, **dadurch gekennzeichnet, dass** diese einen pH-Wert von 6 bis 7,2 aufweist

11. Wässrige pharmazeutische Zubereitung gemäß einem oder mehreren der Ansprüche 1 bis 10, **dadurch gekennzeichnet, dass** diese 20 bis 120 mg/ml Cilengitide und 15 bis 25 Gew.-% Hydroxypropyl-β-Cyclodextrin mit einem molaren Substitutionsgrad von 0,5 bis 0,8 enthält

12. Wässrige pharmazeutische Zubereitung gemäß Anspruch 11, **dadurch gekennzeichnet, dass** diese cirka 80 mg/ml Cilengitide und cirka 20 Gew.-% Hydroxypropyl-β-Cyclodextrin mit einem molaren Substitutionsgrad von cirka 0,58-0,73 enthält

13. Verfahren zur Herstellung einer wässrigen pharmazeutischen Zubereitung gemäß einem oder mehreren der Ansprüche 1 bis 12, **dadurch gekennzeichnet, dass** man zunächst den β-Cyclodextrinether in Wasser löst und anschließend den Wirkstoff sowie ggf. die weiteren Hilfsstoffe zufügt

## Claims

1. Aqueous pharmaceutical preparation of oligopeptides, comprising an oligopeptide of the formula I
cyclo(n-Arg-nGly-nAsp-nD-nE) (I)
in which
D and E each, independently of one another, denote Gly, Ala, β-Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-HalPhe, homoPhe, Phg, Pro, Pya, Ser, Thr, Tia, Tic, Trp, Tyr or Val, where the said amino acid radicals may also be derivatised,
R denotes alkyl having 1-18 C atoms,
Hal denotes F, Cl, Br, I,
Ac denotes alkanoyl having 1-10 C atoms, aroyl having 7-11 C atoms or aralkanoyl having 8-12 C atoms,
n denotes a hydrogen atom or an alkyl radical R, benzyl or an aralkyl radical having 7-18 C atoms on the alpha-amino function of the corresponding amino acid radical,
with the proviso that at least one amino acid radical has a substituent n, where n denotes R,
and where, if they are radicals of optically active amino acids and amino acid derivatives, both the D- and L-forms are encompassed, and physiologically acceptable salts thereof,
and an etherified β-cyclodextrin having a water solubility of greater than 1.8 mg/ml of water.

2. Aqueous pharmaceutical preparation according to Claim 1, **characterised in that** the etherified β-cyclodextrin present is partially etherified β-cyclodextrin.

3. Aqueous pharmaceutical preparation according to Claim 1 or 2, **characterised in that** the ether substituents in the etherified β-cyclodextrin are hydroxyethyl and/or hydroxypropyl groups.

4. Aqueous pharmaceutical preparation according to one or more of Claims 1 to 3, **characterised in that** the etherified β-cyclodextrin has a molar degree of substitution of between 0.2 and 10.

5. Aqueous pharmaceutical preparation according to Claim 4, **characterised in that** the partially etherified β-cyclodextrin has a molar degree of substitution of between 0.2 and 2, based on the ether substituents.

6. Aqueous pharmaceutical preparation according to Claim 4, **characterised in that** the partially etherified β-cyclodextrin has a molar degree of substitution of between 0.5 and 0.8, based on the ether substituents.

7. Aqueous pharmaceutical preparation according to one or more of Claims 1 to 6, **characterised in that** the oligopeptide is cilengitide.

8. Aqueous pharmaceutical preparation according to one or more of Claims 1 to 7, **characterised in that** an isotonic agent is furthermore present in an amount necessary for establishing isotonicity.

9. Aqueous pharmaceutical preparation according to one or more of Claims 1 to 8, **characterised in that** it has a pH of 5 to 8, preferably a pH of 5.6 to 7.4.

10. Aqueous pharmaceutical preparation according to Claim 9, **characterised in that** it has a pH of 6 to 7.2.

11. Aqueous pharmaceutical preparation according to one or more of Claims 1 to 10, **characterised in that** it comprises 20 to 120 mg/ml of cilengitide and 15 to 25% by weight of hydroxypropyl-β-cyclodextrin having a molar degree of substitution of 0.5 to 0.8.

12. Aqueous pharmaceutical preparation according to Claim 11, **characterised in that** it comprises about 80 mg/ml of cilengitide and about 20% by weight of hydroxypropyl-β-cyclodextrin having a molar degree of substitution of about 0.58-0.73.

13. Process for the preparation of an aqueous pharmaceutical preparation according to one or more of Claims 1 to 12, **characterised in that** firstly the β-cyclodextrin ether is dissolved in water, and the active ingredient and any further adjuvants are subsequently added.

## Revendications

1. Préparation pharmaceutique aqueuse d'oligopeptides, comprenant un oligopeptide de la formule I
cyclo(n-Arg-nGly-nAsp-nD-nE) (I)
dans laquelle
D et E chacun indépendamment de l'autre, représentent Gly, Ala, β-Ala, Asn, Asp, Asp(OR), Arg, Cha, Cys, Gln, Glu, His, Ile, Leu, Lys, Lys(Ac), Lys(AcNH₂), Lys(AcSH), Met, Nal, Nle, Orn, Phe, 4-Hal-Phe, homoPhe, Phg, Pro, Pya, Ser, Thr, Tia, Tic, Trp, Tyr ou Val, où lesdits radicaux acides aminés peuvent également être dérivés,
R représente alkyle comportant de 1 à 18 atomes de C,
Hal représente F, CI, Br, I,
Ac représente alkanoyle comportant de 1 à 10 atomes de C, aroyle comportant de 7 à 11 atomes de C ou aralkanoyle comportant de 8 à 12 atomes de C,
n représente un atome d'hydrogène ou un radical alkyle R, un radical benzyle ou un radical aralkyle comportant de 7 à 18 atomes de C concernant la fonction alpha-amino du radical acide aminé correspondant,
étant entendu qu'au moins un radical acide aminé comporte un substituant n, où n représente R,
et où, si ce sont des radicaux d'acides aminés actifs optiquement et des dérivés d'acides aminés actifs optiquement, les formes D- et L- sont toutes deux englobées, et leurs sels physiologiquement acceptables,
et une β-cyclodextrine éthérifiée présentant une solubilité à l'eau supérieure à 1,8 mg/ml d'eau.

2. Préparation pharmaceutique aqueuse selon la revendication 1, **caractérisée en ce que** la β-cyclodextrine éthérifiée présente est une β-cyclodextrine partiellement éthérifiée.

3. Préparation pharmaceutique aqueuse selon la revendication 1 ou 2, **caractérisée en ce que** les substituants d'éther dans la β-cyclodextrine éthérifiée sont des groupes hydroxyéthyle et/ou hydroxypropyle.

4. Préparation pharmaceutique aqueuse selon une ou plusieurs des revendications 1 à 3, **caractérisée en ce que** la β-cyclodextrine éthérifiée présente un degré molaire de substitution entre 0,2 et 10.

5. Préparation pharmaceutique aqueuse selon la revendication 4, **caractérisée en ce que** la β-cyclodextrine partiellement éthérifiée présente un degré molaire de substitution entre 0,2 et 2, sur la base des substituants d'éther.

6. Préparation pharmaceutique aqueuse selon la revendication 4, **caractérisée en ce que** la cyclodextrine partiellement éthérifiée présente un degré molaire de substitution entre 0,5 et 0,8, sur la base des substituants d'éther.

7. Préparation pharmaceutique aqueuse selon une ou plusieurs des revendications 1 à 6, **caractérisée en ce que** l'oligopeptide est cilengitide.

8. Préparation pharmaceutique aqueuse selon une ou plusieurs des revendications 1 à 7, **caractérisée en ce qu'**un agent isotonique est en outre présent selon une quantité nécessaire pour établir une isotonicité.

9. Préparation pharmaceutique aqueuse selon une ou plusieurs des revendications 1 à 8, **caractérisée en ce qu'**elle présente un pH de 5 à 8, de préférence un pH de 5,6 à 7,4.

10. Préparation pharmaceutique aqueuse selon la revendication 9, **caractérisée en ce qu'**elle présente un pH de 6 à 7,2.

11. Préparation pharmaceutique aqueuse selon une ou plusieurs des revendications 1 à 10, **caractérisée en ce qu'**elle comprend de 20 à 120 mg/ml de cilengitide et de 15 à 25% en poids d'hydroxypropyl-β-cyclodextrine présentant un degré molaire de substitution de 0,5 à 0,8.

12. Préparation pharmaceutique aqueuse selon la revendication 11, **caractérisée en ce qu'**elle comprend environ 80 mg/ml de cilengitide et environ 20% en poids d'hydroxypropyl-β-cyclodextrine présentant un degré molaire de substitution d'environ 0,58 à 0,73.

13. Procédé pour la préparation d'une préparation pharmaceutique aqueuse selon une ou plusieurs des revendications 1 à 12, **caractérisé en ce qu'**en premier lieu, l'éther de β-cyclodextrine est dissous dans de l'eau, et l'ingrédient actif et de quelconques adjuvants supplémentaires sont ensuite ajoutés.
